# NEUE EUROPÄISCHE PATENTSCHRIFT

(11) **EP 1 685 854 B2**
(45) Veröffentlichungstag und Bekanntmachung des Hinweises auf die Entscheidung über den Einspruch: **10.06.2020**
(45) Hinweis auf die Patenterteilung: 27.10.2010
(21) Anmeldenummer: 06100915.5
(22) Anmeldetag: 27.01.2006
(51) Int. Cl.: A61L 2/18, A01N 31/02, A01N 59/26, A61L 2/00, A61L 2/22

(54) **Viruzides Desinfektionsmittel**
Virucidal disinfection composition
Composition virucidal en désinfection

(30) Priorität: 28.01.2005 EP 05100562
(43) Veröffentlichungstag der Anmeldung: 02.08.2006
(73) Patentinhaber: B. Braun Medical AG, 6020 Emmenbrücke (CH)
(72) Erfinder: Arndt, Andreas, 6003, Luzern (CH); Willi, Olga, 6207, Nottwill (CH)
(74) Vertreter: dompatent von Kreisler Selting Werner - Partnerschaft von Patent- und Rechtsanwälten mbB

(56) Entgegenhaltungen:
- EP-A- 0 940 145
- WO-A1-2005/051342
- DE-A1- 19 603 977
- JP-A- 2004 189 613
- JP-A- 2004 189 613
- US-A- 5 622 708
- US-A1- 2002 192 297
- US-A1- 2004 146 479
- BA B B ET AL: "Separation methods for antiviral phosphorus-containing drugs" JOURNAL OF CHROMATOGRAPHY B: BIOMEDICAL SCIENCES & APPLICATIONS, ELSEVIER SCIENCE PUBLISHERS, NL, Bd. 764, Nr. 1-2, 25. November 2001 (2001-11-25), Seiten 349-362, XP004322160 ISSN: 1570-0232
- PATENT ABSTRACTS OF JAPAN Bd. 013, Nr. 145 (C-583), 10. April 1989 (1989-04-10) & JP 63 305872 A (SANYO SCOTT KK), 13. Dezember 1988 (1988-12-13)
- EG-Sicherheitsdatenblatt für Materialnummer 1083-Ma-Syn Manorapid Synergy

## Beschreibung

Gegenstand der Erfindung ist ein viruzides Desinfektionsmittel mit Breitbandwirkung, die Verwendung des Desinfektionsmittels zur hygienischen Desinfektion belebter und unbelebter Oberflächen als auch zur Abtötung von Viren, Bakterien und Pilzen.

Weiterhin betrifft die Erfindung ein Desinfektionsverfahren sowie ein Erzeugnis enthaltend ein Desinfektionsmittel.

Desinfektionsmittel haben in den vergangenen Jahren zunehmend an Bedeutung gewonnen. Eine besondere Bedeutung hat der Einsatz von Desinfektionsmitteln in Bereichen wie beispielsweise Krankenhäusern, Arztpraxen, Altenheimen, Unfallstationen, aber auch in Betrieben, die mit der medizinischen Hilfeleistung nichts zu tun haben, wie beispielsweise in Nahrungsmittel herstellenden Betrieben, in der Pharmabranche und in weiteren Branchen, in denen unter Reinstraumbedingungen gearbeitet werden muss, erlangt. Besonders wichtig in diesen Bereichen ist die Desinfektion von Händen, da diese vielfach als Übertragungsweg von Krankheitserregern aller Art verantwortlich sind.

Die Händedesinfektion ist eine anerkannte Methode zur Verhinderung der Übertragung von Infektionen. Dazu werden heute dem Stand der Technik entsprechend Produkte auf alkoholischer Basis zum Einreiben benutzt. Dieses Prinzip ist seit etwa 30 Jahren bekannt. Die zum Einsatz kommenden Präparate basieren in der Regel auf den Wirkstoffen Ethanol, Propan-1-ol, Propan-2-ol bzw. Mischungen der genannten Alkohole.
Mit den bekannten und im Markt erhältlichen Präparaten, welche einen Alkoholanteil von 60 % und mehr aufweisen, gelingt es, die transiente Keimflora von den Händen innerhalb von 30 Sekunden wirksam zu reduzieren.

Die genannten Alkohole und deren Mischungen sowie viele im Handel erhältliche Präparate sind in der Lage, behüllte Viren zu inaktivieren. Sofern man jedoch auf eine umfassende Viruswirksamkeit angewiesen ist, weisen die Alkohole sowie die dem Fachmann bekannten Präparate eine Reihe von Nachteilen auf. So ist bekannt, dass Ethanol erst in hohen Konzentrationen gegen behüllte und unbehüllte Viren wirksam ist. Propan-1-ol und Propan-2-ol sind hingegen nur selektiv wirksam; sie sind unwirksam gegen die meisten unbehüllten Viren, z.B. gegen die sehr resistenten Picornaviren, wozu auch Polio-Viren und das Hepatitis A Virus zählen.

In der Vergangenheit sind mehrere Versuche unternommen worden, die Wirksamkeit alkoholischer Einreibepräparate gegen Viren zu steigern. Folglich sind auch einige Präparate bekannt, für die eine viruzide Wirksamkeit ausgelobt wird.

Die US-A1-2004/0146479 beschreibt Zusammensetzungen zur Händedesinfektion. Eine beispielhafte Zusammensetzung enthält 55 w/v% Ethanol (70 % v/v), 0,1 w/v% Phosphorsäure, 6 w/v% Polyethylenglykol (400), 2w/v% Polyethylenglykol (4000), 0,50 w/v% Propylenglykol und 0,35 w/v Benzylalkohol.

In europäischen Offenlegungsschrift EP-A2-0556546 werden Mittel beschrieben, welche dadurch gekennzeichnet sind, dass sie wenigstens 60 % wenigstens eines Alkohols sowie wenigstens eine Lewis-Säure enthalten. Als Lewis-Säure werden Salze des Aluminiums oder Zinks erwähnt, wobei die Chlorohydrate und Chloride besonders hervorgehoben werden. Die Produkte sollen eine Wirksamkeit gegen das Poliovirus Typ 1 Stamm Mahoney aufweisen. Von Chloriden ist bekannt, dass sie korrosive Eigenschaften haben, man spricht dabei von chloridinduziertem Lochfraß. Analog ist die Offenbarung der deutschen Offenlegungsschrift DE-A1-4205828 zu bewerten.

In der europäischen Offenlegungsschrift EP-A2-0176720 werden Mittel beschrieben, die mindestens 70 % Methanol und/oder Ethanol, 1 - 10 % Glycerol sowie 0,5 - 5 % Rizinusöl enthalten und sich zur Inaktivierung nackter Viren eignen. Der Nachweis wurde ausschließlich mit dem Polio Wildvirus Typ 1 Mahoney geführt.

In der europäischen Offenlegungsschrift EP-A2-0251303 wird ein viruzides Mittel offenbart, welches wenigstens 70 % Ethanol sowie eine kurzkettige Säure enthält. Das Mittel kann zusätzlich Glycerin und Ricinusöl enthalten. Die Anwendungseigenschaften solcher Produkte erwiesen sich wegen des klebrigen Hautgefühls nach dem Auftragen als nicht optimal.

In der deutschen Offenlegungsschrift DE-A1-4221743 werden Mittel beschrieben, die einen niederen Alkohol sowie das Salz einer niederen Carbonsäure enthalten. Auf Basis dieser Lehre formulierte Produkte erwiesen sich als nicht wirksam gegen Papova-Viren, selbst wenn die Einwirkzeit auf 10 Minuten verlängert wurde.

Im Handel erhältlich ist ein Präparat, welches 32,251 g Propan-1-ol, 20,985 g, Propan-2-ol, 4,2 g Chlorhexidindigluconat-Lösung 20 %, 1-Tetradecanol, Macrogol 4000, Cetearyloctanoat, Patentblau V, Geruchsstoffe und gereinigtes Wasser enthält. Das Präparat ist nach Angaben des Herstellers wirksam gegen, Vaccinia-Viren, Rota-Viren, Hepatitis B Viren, Hepatitis C Viren und HI-Viren.

Ein in der Bundesrepublik Deutschland erhältliches Präparat enthält 95 % Ethanol als Wirkstoffbasis sowie Vergällungsmittel und hautpflegende Substanzen. Das Produkt ist in der Liste des Robert Koch Institutes (RKI) als wirksam gegen Viren eingestuft. Die Einwirkzeit nach Aussage des Herstellers beträgt 2 Minuten. Bestimmend für die Wirksamkeit ist die Einwirkzeit gegen Papova- und Adeno-Viren. Gegen Polioviren ist das Präparat innerhalb von 60 Sekunden wirksam. Das Präparat ist unzureichend gegen Parvoviren wirksam.

Der Flammpunkt des Präparates beträgt nach Herstellerangaben 0°C. Lagerung und Transport eines solchen Präparates sind entsprechend anspruchsvoll.

Die deutsche Patentschrift DE-C1-4424325 bzw. die europäische Offenlegungsschrift EP-A1-0692192 beschreiben eine alkoholische Desinfektionsmittelzubereitung mit einem Gehalt von Ethanol und/oder Methanol von wenigstens 80 Gewichtsprozent, gekennzeichnet durch einen Gehalt an Butanon. Die Zubereitung kann als Remanenzwirkstoff Chlorhexidin oder Benzalkoniumchlorid enthalten. Als Pflegestoffe kann die Formulierung ein Alkylenglykol, namentlich Triethylenglykol oder Glycerin sowie einen Ester einer langkettigen Fettsäure enthalten. Die Desinfektionsmittelzubereitung wird zur Inaktivierung von unbehüllten Viren, insbesondere von Polioviren beansprucht.

Die deutsche Offenlegungsschrift DE-A1-19962353 beschreibt ein Hepatitis A abtötendes Mittel, welches einen oder mehrere Alkohole und bis zu 0,5 % eines chlorhaltigen oder chlorabspaltenden Mittels enthält. Das Mittel kann weiterhin bis zu 10 % antimikrobielle Säuren wie Undecylensäure, Citronensäure, p-Hydroxybenzoesäure, Sorbinsäure, Salicylsäure enthalten. Im Markt erhältlich ist ein Präparat, welches 90 % Ethanol, 0,2 % Chlorhexidingluconat sowie weitere Zusätze enthält. Dieses Präparat basiert offenbar auf der deutschen Patentschrift DE-C1-4424325 und/oder der deutschen Offenlegungsschrift DE-A1-19962353. Das im Markt erhältliche Präparat weist einen niedrigen Flammpunkt auf, den der Hersteller mit 15°C angibt. Eine Wirksamkeit dieses Präparats gegen Papova-Viren ist nicht bekannt.

In der veröffentlichten internationalen Patentanmeldung WO-A1-97/35475 wird ein Produkt mit einem Flammpunkt von über 21 °C offenbart, welches niedere Alkohole und Synergisten enthält. Als Synergisten werden Diole, speziell Propylenglykol, Butylenglykol und deren Mischungen eingesetzt. Das Produkt kann eine physiologisch unbedenkliche organische Säure enthalten. Ein auf dieser Patentschrift basierendes Handelsprodukt enthält 54,1 % Ethanol, 10 % 1-Propanol, 5,9 % Propan-1,2-diol und 5,7 % Butan-1,3-diol. Für die kurzzeitige Anwendung scheint das Produkt zur hygienischen Händedesinfektion geeignet zu sein. Bei Dauergebrauch erweist sich jedoch der hohe Anteil der nichtflüchtigen Diole als störend, da diese als Rückstände auf den Händen verbleiben und ein unangenehmes Hautgefühl verursachen können. Speziell für die Anwendung zur chirurgischen Händedesinfektion sind Präparate gemäss WO-A1-97/35475 nur eingeschränkt geeignet.

In der europäischen Offenlegungsschrift EP-A1-0848907 wird ein Spraydesinfektionsmittel beansprucht, welches 30 bis 70 % Ethanol, eine Aminkomponente sowie einen Terpenkohlenwasserstoff enthält. Damit lassen sich Präparate mit Wirksamkeit gegen Polioviren formulieren. Aufgrund der Zusammensetzung können Produkte dieser Art jedoch nur zur Desinfektion unbelebter Oberflächen eingesetzt werden.

In der deutschen Offenlegungsschrift DE-A1-10237227 wird ein alkoholisches Hände- und Hautdesinfektionsmittel mit Ascorbinsäure und/oder deren Abbauprodukten beschrieben, welches eine verbesserte Wirksamkeit gegen Poliovirus Typ 1 Stamm Mahoney und Adenovirus Stamm Adenoid 6 aufweisen soll. Eines der Abbauprodukte ist die physiologisch nicht unbedenkliche Oxalsäure.

Für Persäuren ist eine Wirksamkeit gegen Bakterien und Viren bekannt, so dass es logisch erscheint, Persäuren auch für die Desinfektion der Hände einzusetzen. In der RKI-Liste ist dazu ein Präparat auf Basis verdünnter wässriger Peressigsäure aufgeführt, welches aber nicht als wirksam gegen Viren eingestuft wurde.

In der deutschen Offenlegungsschrift DE-A1-19724102 werden Mittel zur Schnelldesinfektion oder Dekontamination enthaltend einen Gehalt an physiologisch verträglichen Persäuren beschrieben. Es ist nicht bekannt, ob das Produkt auch wirksam gegen Viren ist. Unabhängig davon ist das Handling aufwändig, die Stabilität der zur Anwendung gelangenden Gebrauchslösungen ist eingeschränkt.

In der deutschen Offenlegungsschrift DE-A1-10106444 werden Mittel zur viruziden Händedesinfektion beansprucht, die aus den Komponenten A und B hergestellt werden, wobei die Komponente A aus 50 bis 80 % eines flüssigen aliphatischen Alkohols oder Mischungen von flüssigen aliphatischen Alkoholen besteht und Komponente B eine 20 bis 30 %ige Lösung aus Glycerinmonoperacetatdiacetat und/oder Glycerinmonoperacetatacetat und/oder Glycolmonoperacetatacetat sowie Harnstoff umfasst. Die Komponenten A und B müssen in einer Dosier- und Mischvorrichtung vereinigt werden. Die Komponenten A und B sollen stabil sein, die Mischung der Komponenten A und ist jedoch nicht über längere Zeit lagerstabil. Zudem erfordert die Mischung der Komponenten einen höheren logistischen Aufwand, um z.B. der Verwechslungsgefahr der Komponenten vorzubeugen. Ebenso ist die Dosier- und Mischvorrichtung so einzurichten, dass ein vorzeitiges Aufbrauchen einer der Komponenten in jedem Falle vermieden wird.

Anorganische Säuren haben meist nur eine beschränkte Viruswirksamkeit. In dem vom Springer-Verlag Berlin Heidelberg New York im Jahre 2002 herausgegebenen Handbuch der viruswirksamen Desinfektion (ISBN 3-540-67532-9) heben die Autoren auf Seite 79 hervor, dass manche Viren, die im Verlauf ihres Infektionsweges den Magen passieren müssen, gegenüber Säuren recht resistent sind. Die Autoren betonen, dass Phosphorsäure auch noch in einer Konzentration von 3 % unwirksam gegen Poliovirus ist.

Die Deutschen Gesellschaft für Hygiene und Mikrobiologie (DGHM) hatte in der Vergangenheit in ihrer Liste Präparate zur Instrumentendesinfektion für viruzid erklärt, wenn eine Wirksamkeit gegen das Poliovirus Typ 1 Stamm Mahoney nachgewiesen werden konnte. Eine generelle Viruzidie kann aus der Poliowirksamkeit allein jedoch nicht abgeleitet werden. Andererseits ist es weder möglich noch sinnvoll, die Wirksamkeit gegen Viren anhand aller bekannten Viren einzeln prüfen zu wollen. Dies ist derzeit bereits methodisch nicht möglich, da trotz des voranschreitenden technischen Fortschritts für eine Reihe von Viren bislang keine Möglichkeit der in vitro Anzucht gefunden wurde.

Bei der Ermittlung der Viruzidie hat es sich deshalb als zweckmässig erwiesen, die Wirksamkeit anhand ausgewählter kritischer Modellviren zu ermitteln, um auf Basis dieser Ergebnisse dann einen Rückschluss auf die allgemeine Viruzidie zu ziehen. Dieses Vorgehen wird durch die Richtlinie des Bundesgesundheitsamtes und der Deutschen Vereinigung zur Bekämpfung der Viruskrankheiten aus dem Jahre 1982 gestützt.
Aktualisiert wurde diese Richtlinie durch die im Jahre 2004 erschienene Stellungnahme des Arbeitskreises Viruzidie beim Robert Koch-Institut (RKI) sowie des Fachausschusses "Virusdesinfektion" der Deutschen Gesellschaft zur Bekämpfung der Viruskrankheiten (DVV) und der Desinfektionsmittelkommission der Deutschen Gesellschaft für Hygiene und Mikrobiologie (DGHM).
In dieser Stellungnahme wird die Prüfung und Deklaration der Wirksamkeit von Desinfektionsmitteln gegen Viren in Form einer RKI-Empfehlung neu geregelt. Die RKI-Empfehlung ist in der Fachzeitschrift Bundesgesundheitsblatt - Gesundheitsforschung - Gesundheitsschutz 2004, 47, S. 62-66 erschienen.
Diese Empfehlung wird im folgenden Text verkürzt als "RKI-Viruzidie-Empfehlung" bezeichnet.
Gemäss dieser RKI-Viruzidie-Empfehlung werden in der Bundesrepublik Deutschland Präparate mit dem Anspruch auf viruzide Wirksamkeit gegen folgende Modellviren geprüft: Adenovirus Typ 5, Stamm Adenoid 75), Papovavirus [Simianvirus 40 (SV40), Stamm 777], Poliovirus (Polio-Impfstamm Typ I, Stamm LSc-2ab), Vacciniavirus (Stamm Elstree). Von einer Wirksamkeit gegen das jeweilige Prüfvirus wird dann ausgegangen, wenn im quantitativen Suspensionsversuch eine Reduktion des Virustiters von mindestens 4 Logarithmus Stufen erreicht wird.
Bei der Viruzidieprüfung werden 8 Teile Produkt, 1 Teil Virussuspension und 1 Teil Belastungssubstanz bzw. Wasser vermischt. Methodisch bedingt ergibt sich damit eine 80 %ige Konzentration des Prüfpräparates. Die Prüfpräparate werden ohne Belastung, bei Belastung mit 0,2 % Rinderalbumin und mit 10 % fötalem Kälberserum geprüft. Nur ein Präparat, welches unter allen Prüfbedingungen eine Reduktion des Virustiters von mindestens 4 Logarithmus Stufen erreicht, erfüllt die Vorgaben des RKI hinsichtlich Virus-Wirksamkeit. In der Europäischen Prüfnorm EN 14476, in welcher der quantitative Suspensionsversuch auf Viruzidie in der Humanmedizin beschrieben wird, wird unter Punkt 1 darauf verwiesen, dass ein Desinfektionsmittel oder Antiseptikum, das in unverdünnter Form verwendet wird, in 80 %iger Konzentration geprüft werden muss.
Erreicht ein Prüfpräparat die geforderte Wirksamkeit gegen das Prüfvirus nicht, so hat es sich etabliert, dass der Hersteller sein Präparat aufkonzentriert, in dem er auf einen Teil des normalerweise enthaltenen Wassers in seiner Rezeptur verzichtet - und ein so speziell aufbereitetes Präparat noch einmal in einer höheren Konzentration nachprüft. So sind in der Literatur Angaben über Prüfpräparate zu finden, bei denen zwar eine Viruswirksamkeit innerhalb von 1 bis 2 Minuten ausgelobt wird, die Konzentration der Prüfpräparate jedoch mit z.B. 90, 94 und 100 % dokumentiert ist. Streng genommen ist dies eine Abweichung von den Standardprüfbedingungen und solche Präparate sind damit eigentlich nicht als viruzid entsprechend der RKI-Viruzidie-Empfehlung sowie der Europäischen Prüfnorm EN 14476 einzustufen. Zudem wird die objektive Vergleichbarkeit der Präparate untereinander erschwert.

Vergleicht man die Richtlinie des Bundesgesundheitsamtes und der Deutschen Vereinigung zur Bekämpfung der Viruskrankheiten aus dem Jahre 1982 mit der aktuellen RKI-Viruzidie-Empfehlung, so fällt auf, dass einzelne Prüf-Viren aus methodischen Gründen geändert wurden. So wurde statt des Polio-Impfstammes Typ I, Stamm LSc-2ab früher der Wildvirus Typ 1 Stamm Mahoney verwendet. Analog sieht es mit dem Adeno-Virus aus, wo früher der Stamm Adenoid 6 verwendet wurde. Die Änderungen bei den Virusstämmen zeigten interessante Ergebnisse auf. Ein geringfügig abgewandeltes Produkt, basierend auf den Wirkstoffen 78,2 % Ethanol 96 %ig und 10 % 2-Propanol , welches gegen den Poliovirus Typ 1 Stamm Mahoney eine Wirksamkeit innerhalb von 2 Minuten erreicht hatte, erwies sich gegenüber dem Polio-Impfstamm Typ I, Stamm LSc-2ab als nicht ausreichend wirksam. Es ist deshalb zu hinterfragen, ob die in der Vergangenheit ermittelten Ergebnisse zur Wirksamkeit von Präparaten gegenüber dem Poliovirus Typ 1 Stamm Mahoney generell auch auf den Polio-Impfstamm Typ I, Stamm LSc-2ab zu übertragen sind.

Ein anderes Problem offenbart sich bei der praktischen Durchführung der Händedesinfektion. In der Regel halten medizinische Einrichtungen heute mehrere Präparate vorrätig: Ein Produkt für die Routinedesinfektion sowie ein weiteres Präparat speziell zur Anwendung bei unbekannter Virusexposition. Die sich daraus ableitenden logistischen Schwierigkeiten sowie der hohe Schulungsbedarf des Personals stellen eine nicht zu unterschätzende Herausforderung dar.
Es wird festgestellt, dass auch heute noch ein Bedarf an verbesserten Desinfektionsmitteln, speziell Händedesinfektionsmitteln mit viruzider Wirksamkeit besteht, insbesondere an solchen mit Breitbandwirkung, die sowohl für den Routinegebrauch als auch bei unbekannter Virusexposition geeignet sind.

Aufgabe der vorliegenden Erfindung war es nun, ein Desinfektionsmittel zur Verfügung zu stellen, das neben einer bakteriziden und fungiziden Wirksamkeit vor allem eine verbesserte und umfassende Wirksamkeit gegen Viren aufweist und zu dem im Dauergebrauch bei Kontakt mit menschlicher Haut gut verträglich ist.

Überraschend wurde nun gefunden, dass viruzide Desinfektionsmittel, die Phosphorverbindungen, Alkalimetallsalze der Phosphorsäure, alkoholische Komponenten und Polyalkylenglycol enthalten den zuvor erwähnten Anforderungen entsprechen.

In einer ersten allgemeinen Ausführungsform ist der Gegenstand der vorliegenden Erfindung daher ein viruzides Desinfektionsmittel mit Breitbandwirkung umfassend
a) eine oder mehrere saure Phosphorverbindung(en), die ausgewählt ist/sind aus der Gruppe der Phosphorsäure, Diphosphorsäure, Triphosphorsäure, Polyphosphorsäure der allgemeinen Formel Hₙ₊₂PₙO₃ₙ₊₁ mit n gleich eine ganze Zahl von 1 bis 17, cyclo-Tri- und Tetrametaphosphorsäure, Polymet-phosphorsäure, Peroxomonophosphorsäure, Peroxodiphosphorsäure, Hypophosphorsäure, Diphosphor(III, IV)-säure, sowie der Salze dieser Säuren, in einer Menge von 0,2 bis 1,5 Gew.-%, bezogen auf das Mittel, mit der Maßgabe, dass die sauren Phosphorverbindungen Phosphorsäure und ein oder mehrere Alkalimetallsalze der Phosphorsäure umfasst und die Phosphorsäure und die ein oder mehreren Alkalimetallsalze der Phosphorsäure in einer Menge von 0,2 bis 1,5 Gew.-%, bezogen auf das Mittel, vorliegen,
b) als alkoholische Komponente Ethanol und/oder Propan-1-ol und/oder Propan-2-ol, vorzugsweise Mischungen aus Ethanol und Propon-1-ol, in einer Menge von 30 bis 80 Gew.-%, bezogen auf das gesamte Mittel, und
c) einen oder mehrere Polyalkylenglykol(e), der allgemeinen Formel HO-[R-O]ₙ-H, in der R für (CH₂)₂, CH₂CH(CH₃), CH₂CH(CH₂CH₃) oder für (CH₂)₄ und n für Werte von 2 bis 200 steht, enthält.

Die erfindungsgemäßen viruziden Desinfektionsmittel enthalten als wesentlichen Bestandteil eine oder mehrere saure Phosphorverbindung(en) und ein oder mehrere Alkalimetallsalze der Phosphorsäure. Phosphorverbindungen, die in den erfindungsgemäßen Desinfektionsmitteln zum Einsatz kommen, sind saure Phosphorverbindungen und deren Salze. Saure Phosphorverbindungen im Sinne der vorliegenden Erfindung sind Phosphorverbindungen, die im wässrigen Milieu in der Lage sind, Protonen abzugeben (Brönstedt-Säuren). Eingesetzt werden Phosphorsäure sowie die Salze der vorgenannten Säure. Bei den Salzen handelt es sich um die Alkalimetallsalze, insbesondere die Natrium- und Kaliumsalze der genannten sauren Phosphorverbindungen.

An die sauren Phosphorverbindungen und deren Salze werden darüber hinaus keine besonderen Anforderungen gestellt. Es ist jedoch vorteilhaft, dass sowohl die saure Phosphorverbindung als auch die Salze der sauren Phosphorverbindungen in den vorzugsweise wässrig-alkoholischen Desinfektionsmittelzusammensetzungen löslich sind.

Phosphorsäure und ein oder mehrerer Alkalimetallsalze der Phosphorsäure bilden einen wesentlichen Bestandteil des erfindungsgemäßen Desinfektionsmittels. Als besonders geeignet hat sich der Einsatz von Phosphorsäure und Natriumdihydrogenphosphat und/ oder Kaliumdihydrogenphosphat herausgestellt. Zudem wurde überraschend gefunden, dass durch Mischungen aus Phosphorsäure mit Natriumdihydrogenphosphat und/oder Kaliumdihydrogenphosphat die Hautverträglichkeit erheblich heraufgesetzt werden konnte. Dies hat insbesondere für den Einsatz des Desinfektionsmittels als Händedesinfektionsmittel Bedeutung.

Ein oder mehrere Phosphorverbindungen werden in einer Menge von 0,2 bis 1,5 Gewichtsprozent, vorzugsweise 0,2 bis 1,0 Gewichtsprozent und insbesondere von 0,2 bis 0,8 Gewichtsprozent, jeweils bezogen auf das gesamte Mittel, eingesetzt.

Als weitere wesentliche Komponente enthalten die erfindungsgemäßen Desinfektionsmittel einen oder mehrere Polyalkylenglykole der allgemeinen Formel HO-[R-O]ₙ-H, In der R für (CH₂)₂, CH₂CH(CH₃), CH₂CH(CH₂CH₃) oder für (CH₂)₄ und n für Werte von 2 bis 200 steht.

Polyalkylenglykole (Polyglykole, Polyglykolether; INCI Chemical Class: Polymeric Ethers) sind bekannte, überwiegend lineare, z. T. aber auch verzweigte Polyether, bei denen es sich um Polymere mit endständigen Hydroxy-Gruppen handelt. Die Polyalkylenglykole mit höheren Molmassen sind polymolekular, d. h. sie bestehen aus Kollektiven von Makromolekülen mit unterschiedlichen Molmassen.

Die mittleren relativen Molmassen der Polyalkylenglykole liegen vorzugsweise im Bereich von 400 bis 10000, insbesondere von 1000 bis 8000, besonders bevorzugt von 2000 bis 6000 und äußerst bevorzugt von 3000 bis 5000. Bei den nachfolgend beschriebenen verschiedenen Polyalkylenglykolen können bestimmte Bereiche nochmals besonders vorteilhaft sein.

Erfindungsgemäß bevorzugt sind lineare oder verzweigte, insbesondere lineare Polyalkylenglykole der allgemeinen Formel HOIR-OL-H, in der R für (CH₂)₂, CH₂CH(CH₃), CH₂CH(CH₂CH₃), und/oder für (CH₂)₄ und n für Werte bzw. Mittelwerte von 2 bis etwa 200, vorzugsweise 3 bis 190, weiter bevorzugt 4 bis 180, besonders bevorzugt 6 bis 150, insbesondere 10 - 120 stehen. Die Polyalkylenglykole können durch ringöffnende Polymerisation von Ethylenoxid, Propylenoxid und/oder Tetrahydrofuran hergestellt werden. Es sind dies im einzelnen die Polyethylenglykole mit R = (CH2)2, die Polypropylenglykole mit R = CH₂CH(CH₃), die Polytetrahydrofurane mit R = (CH₂)₄ und die Copolymere aus Ethylenoxid, Propylenoxid und/oder Tetrahydrofuran.

Erfindungsgemäß bevorzugt sind Polyethylenglykole (PEG) mit einer mittleren relativen Molmasse von 400 bis 10000, insbesondere von 1000 bis 8000, besonders bevorzugt von 2000 bis 6000 und äußerst bevorzugt von 3000 bis 5000. Für Polyethylenglykole existieren verschiedene Nomenklaturen, die zu Verwirrungen führen können. Technisch gebräuchlich ist die Angabe des mittleren relativen Molgewichts im Anschluss an die Angabe "PEG", so dass "PEG 200" ein Polyethylenglykol mit einer relativen Molmasse von ca. 190 bis ca. 210 charakterisiert. Gemäß INCI-Nomenklatur wird das Kurzzeichen PEG mit einem Bindestrich versehen und folgt direkt auf den Bindestrich eine Zahl, die der Zahl n in der obigen allgemeinen Formel entspricht. Kommerziell erhältliche Polyethylenglykole sind beispielsweise PEG 200/PEG-4, PEG 300/PEG-6, PEG- 7, PEG-8, PEG 400, PEG-9, PEG-10, PEG-12, PEG 600, PEG-14, PEG-16, PEG 800/PEG-18, PEG-20, PEG 1000, PEG 1200, PEG 1500/PEG-32, PEG-40, PEG 2000, PEG-55, PEG-60, PEG 3000, PEG 3350/PEG-75 und PEG 4000/PEG-90, wobei die Bezeichnungen gemäß den beiden Nomenklaturen für einander entsprechende Polyethylenglykole durch das Zeichen "1" getrennt nebeneinander gestellt sind. Die kommerziell erhältlichen Polyethylenglykole sind beispielsweise unter den Handelsnamen Carbowax® (Union Carbide), Emkapol® und Reflex® PEG (ICI), Lipoxol® (DEA), Polyglykol® E (Dow), Pluracol® E, Pluriol® E sowie Lutrol® E (BASF) verfügbar.

Polypropylenglykole (PPG) sind über einen breiten Molmassenbereich von 250 (PPG-4) bis 4.000 (PPG-69) klare, nahezu farblose Flüssigkeiten, für deren Bezeichnung die zuvor beschriebene INCI-Nomenklatur analog verwendet wird. So werden die Polypropylenglykole der obigen allgemeinen Formel mit Werten n von 5 bzw. 6 als PEG-5 bzw. PEG-6 bezeichnet. Die niedermolekularen Polypropylenglykole sind mit Wasser mischbar, während die höhermolekularen Vertreter weniger wasserlöslich sind. Kommerziell erhältlich sind beispielsweise die gemäß INCI bezeichneten Polypropylenglykole PPG-7, PPG-9, PPG-12, PPG-13, PPG-15, PPG-17, PPG-20, PPG-26, PPG-30, PPG-33, PPG-34, PPG-51 und PPG-69. Bezugsquellen sind dem International Cosmetic Ingredient Dictionary and Handbook zu entnehmen.

Bei den Copolymeren handelt es sich vorzugsweise um statistische Copolymere und insbesondere um Blockcopolymere aus Ethylen- und Propylenoxid, Ethylenoxid und Tetrahydrofuran, Propylenoxid und Tetrahydrofuran oder Ethylenoxid, Propylenoxid und Tetrahydrofuran, bevorzugt aus Ethylen- und Propylenoxid, besonders bevorzugt um Blockcopolymere aus Ethylen- und Propylenoxid.

Erfindungsgemäß bevorzugte statistische Copolymere aus a Ethylen- und b Propylenoxideinheiten sind beispielsweise folgende gemäß International Cosmetic Ingredient Dictionary and Handbook als PEG/PPG-a/b bezeichnete Copolymere (Molmasse), wobei a und b Mittelwerte darstellen: PEG/PPG-18/4 Copolymer (1000), PEG/PPG-17/6 Copolymer (1100), PEG/PPG-35/9 Copolymer (2100) und PEG/PPG-23/50 Copolymer (3900).

Erfindungsgemäß bevorzugte Blockcopolymere aus Ethylen- und Propylenoxid genügen der Formel HO(CH₂CH₂O)ₓ(CH(CH₃)CH₂O)_{y}(CH₂CH₂O)_{x'}H in der x und x' für Mittelwerte von 2 bis 130 und y für Mittelwerte von 15 bis 67 stehen, und werden mit dem internationalen Freinamen Poloxamer bezeichnet, der auch im International Cosmetic Ingredient Dictionary and Handbook verwendet wird. Jedes Poloxamer ist durch eine dreistellige Nummer gekennzeichnet. Die ersten beiden Ziffern geben multipliziert mit 100 die durchschnittliche Molmasse des Polypropylenglykol-Anteils und die letzte Ziffer multipliziert mit 10 den Polyethylenglykol-Anteil in Gew.-% an. Dieser beträgt 10 bis 80 Gew.- %, vorzugsweise nicht mehr als 50 Gew.-%, insbesondere nicht mehr als 40 Gew.-%, besonders bevorzugt nicht mehr als 30 Gew.-%, beispielsweise 10, 20 oder 30 Gew.-%. Die Herstellung der Poloxamere erfolgt zweistufig, wobei zunächst Propylenoxid kontrolliert an Propylenglykol addiert und der erhaltene Polypropylenglykolblock durch anschließende Addition von Ethylenoxid von zwei Polyethylenglykol-Blöcken eingefasst wird. Besonders bevorzugte Blockcopolymere sind beispielsweise folgende flüssige Poloxamer-Typen (x, y, x'; Molmasse; z. T. Schmelzpunkt): Poloxamer 101 (2, 16, 2; 1100; -32), Poloxamer 122 (5, 21, 5; 1630; -26), Poloxamer 123 (7, 21, 7; 1900; -1), Poloxamer 105 (11, 16, 11; 1850; 7), Poloxamer 181 (3, 30, 3; 2000; -29), Poloxamer 124 (11, 21, 11; 2200; 16), Poloxamer 182 (8, 30, 8; 2500; -4), Poloxamer 183 (10, 30, 10; 2650; 10), Poloxamer 212 (8, 35, 8; 2750; -7), Poloxamer 231 (6, 39, 6; 2750; -37), Poloxamer 184 (13, 30, 13; 2900; 16), Poloxamer 185 (19, 30, 19; 3400), Poloxamer 282 (10, 47, 10; 3650; 7), Poloxamer 331 (7, 54, 7; 3800; -23), Poloxamer 234 (22, 39, 22; 4200; 18), Poloxamer 401 (6, 67, 6; 4400; 5), Poloxamer 284 (21, 47, 21; 4600) und Poloxamer 402 (13, 67, 13; 5000; 20). Kommerziell sind die Poloxamere unter den Handelsnamen Pluronic® und Synperonic® PE erhältlich, den ein Buchstabe aus der Gruppe L, P und F sowie eine zwei- oder dreistellige Zahl nachgestellt ist. Dabei ist die letzte Ziffer mit der letzten Ziffer der Poloxamer-Nomenklatur identisch und ergeben die davorstehenden ein- oder zweistelligen Zahlen multipliziert mit 300 die ungefähre Molmasse des Polypropylenglykol-Anteils bzw, mit 3 multipliziert in etwa die aus den ersten beiden Ziffern der Poloxamer-Nomenklatur-Nummer gebildeten Zahl, d. h. entsprechen 3, 4, 6, 7, 8, 9, 10 und 12 in dieser Reihenfolge den zweistelligen Zahlen 10, 12, 18, 21, 23, 28, 33 und 40 am Anfang der Nummer gemäß der Poloxamer-Nomenklatur. Die Buchstaben unterscheiden flüssige (L), pastöse (P) und feste (F) Poloxamere. So ist beispielsweise das Poloxamer 101 als Pluronic^{®} L 31 und Synperonic^{®} PE L 31 erhältlich.

Eine weitere Klasse geeigneter Blockcopolymere aus Ethylen- und Propylenoxid genügen der Formel HO(CH(CH₃)CH₂O)_{y}(CH₂CH₂O)ₓ(CH₂CH(CH₃)O)_{y'}H. Hier wird ein Polyethylenglykol-Block von zwei Polypropylenglykol-Blöcken eingerahmt, während bei den Poloxameren ein Polypropylenglykol-Block von zwei Polyethylenglykol-Blöcken eingefasst wird. Die Herstellung erfolgt wiederum zweistufig, wobei zunächst Ethylenoxid kontrolliert an Ethylenglykol addiert und der erhaltene Polyethylenglykol-Block durch anschließende Addition von Propylenoxid von zwei Polypro-pylenglykol-Blöcken eingefasst wird. Kommerziell erhältlich sind diese Blockcopolymere wie die Poloxamere unter dem Handelsnamen Pluronic^{®} (BASF), dem jeweils ein alphanumerischer Code aus drei Ziffern und dem zwischen die zweite und dritte Ziffer geschobenen Buchstaben R folgt. Die Bedeutung der Ziffern ist mit der Bedeutung im Rahmen der Poloxamer-Nomenklatur identisch. Der eingeschobene Buchstabe R (für engl. reverse) deutet auf die im Vergleich zu den Poloxameren invertierte Struktur hin. Bevorzugte Vertreter dieser Klasse sind die folgenden Pluronic^{®}-Typen (Mol-masse; Schmelzpunkt): Pluronic^{®} 10R5 (1950; 15), Pluronic^{®} 12R3 (1800; -20), Pluronic^{®} 17R1 (1900; -27), Pluronic^{®} 17R2 (2150; -25), Pluronic^{®} 17R4 (2650; 18), Pluronic^{®} 25R1 (2700; -5), Pluronic^{®} 25R2 (3100; - 5), Pluronic^{®} 31R1 (3250; -25) und Pluronic^{®} 31R2 (3300; 9).

Die Polyalkylenglykole werden vorteilhafter Weise in einer Menge von 0,05 bis 8 Gewichtsprozent, vorzugsweise 0,1 bis 5 Gewichtsprozent, besonders bevorzugt von 0,5 bis 3 Gewichtsprozent und insbesondere von 0,8 bis 1,5 Gewichtsprozent, jeweils bezogen auf das gesamte Mittel, eingesetzt.

Als weiteren wesentlichen Bestandteil enthalten die erfindungsgemäßen Desinfektionsmittel wenigstens eine alkoholische Komponente. Vorzugsweise werden aliphatische Alkohol mit einer linearen oder verzweigten Alkylgruppe, die 1 bis 6 Kohlenstoffatome trägt, eingesetzt. Bevorzugt handelt es sich bei den Alkoholen um Mono-Alkohole. Besonders bevorzugt sind aliphatische Alkohole mit 1 bis 4, insbesondere mit 2 bis 3 Kohlenstoffatomen. Besonders stark viruzide Desinfektionsmittel können erhalten werden, wenn als alkoholische Komponente Ethanol und/oder Propan-1-ol und/oder Propan-2-ol, vorzugsweise Mischungen aus Ethanol und Propan-1-ol, eingesetzt werden.

Gerade im Zusammenspiel mit den sauren Phosphorverbindungen, insbesondere der Phosphorsäure, hat sich überraschend gezeigt, dass trotz des sauren pH-Wertes mit den alkoholischen Komponenten eine hervorragende Hautverträglichkeit erzielt werden konnte. Dies war insofern überraschend, da alkoholische Produkte mit einem tendenziell sauren pH-Wert bislang immer als schlecht hautverträglich eingestuft wurden.

Die erfindungsgemäßen Desinfektionsmittel enthalten die alkoholische Komponente in einer Menge von 30 bis 80 Gewichtsprozent, vorzugsweise von 45 bis 80 Gewichtsprozent und insbesondere von 60 bis 75 Gewichtsprozent, jeweils bezogen auf das gesamte Mittel.

Der pH-Wert der unverdünnten Desinfektionsmittel liegt vorzugsweise in einem Bereich von 3 bis 7, weiter bevorzugt 3,5 bis 6,5 und insbesondere zwischen 4 und 6.

Das erfindungsgemäße Desinfektionsmittel weist in einer bevorzugten Ausführungsform einen Flammpunkt (gemessen nach DIN 51755) von wenigstens 21 °C auf. Der hohe Flammpunkt des Desinfektionsmittels erleichtert dabei erheblich den Transport und die Lagerung.

Die erfindungsgemäßen Desinfektionsmittel eignen sich insbesondere für die Desinfektion von belebten Oberflächen, wie beispielsweise Haut und insbesondere die Hände. Es ist daher vorteilhaft, dass die erfindungsgemäßen Desinfektionsmittel zusätzlich Hautpflegekomponenten enthalten. Diese sind vorzugsweise ausgewählt aus der Gruppe bestehend aus Glycerin, Propan-1,2-diol, Butan-1,3-diol, Sorbitol, Dexpanthenol, Allantoin, Bisabolol, Tocoferylacetat, Dodecanol, Tetradecanol, Hexadecanol, Octadecanol, Lanolinalkohol, Cetearylalkohol, Cyclomethicone, Dimethicone, Isopropylmyristat, Isopropylpalmitat, Cetearylethylhexanoat, Octylstearat, Octyloctanoat, Ethylhexansäure, Ethylester, Jojobaöl, Sanddornöl, Wollwachs, Parafinöl, Vaseline, Heptamethylnonan/Isohexadecan, Cholesterol, Partialglyceriden und -triglyceriden.

Bevorzugt enthalten die erfindungsgemäßen Desinfektionsmittel 0,01 bis 5 Gewichtsprozent, insbesondere 0,1 bis 2 Gewichtsprozent, jeweils bezogen auf das gesamte Mittel, einer oder mehrerer Hautpflegekomponente(n).

Insbesondere dann, wenn die erfindungsgemäßen Desinfektionsmittel im Dauergebrauch eingesetzt werden, wie beispielsweise bei Einsatz des Desinfektionsmittels als Händedesinfektionsmittel im klinischen oder pharmazeutischen Bereich, hat sich der Zusatz von Rückfettern als besonders vorteilhaft erwiesen. Rückfettend wirkende Stoffe sorgen dafür, dass Haut, die mit den Desinfektionsmitteln in Berührung kommt, nicht austrocknet. Rückfetter machen die Haut weich und geschmeidig. Rückfetter sind dem Fachmann geläufig. Als besonders geeignet haben sich die Rückfetter erwiesen, die ausgewählt sind aus der Gruppe bestehend aus Glycerintri-, Glycerindi- und Glycerinmonooleat, Glycerylcaprylat, Glycerylcaprat, Polyglyceryl-2-caprat sowie langkettigen linearen oder verzweigten ein- oder mehrwertigen Fettalkoholen, beispielsweise Octyldodecanol. Gleichermaßen wird den Komponenten Isopropylmyristat und Cetearyloctanoat eine rückfettende Wirkung zugeordnet.

Die Rückfetter werden üblicherweise in Mengen von 0,01 bis 5 Gewichtsprozent, vorzugsweise von 0,1 bis 2 Gewichtsprozent, jeweils bezogen auf das gesamte Mittel, eingesetzt.

Zur Unterstützung der Wirksamkeit der erfindungsgemäßen Desinfektionsmittel können diese darüber hinaus weitere Zusatzkomponenten mit mikrobizider Wirkung enthalten. Vorzugsweise sind diese Zusatzkomponenten flüchtig und insbesondere ausgewählt aus der Gruppe bestehend aus Benzalkoniumchlorid, Didecyldimethylammoniumchlorid, Mecetroniumetilsulfat, Octenidin, Polyhexamethylenbiguanid, Chlorhexidingluconat, Chlorhexidinacetat, Cetrimid, Cetylpyridiniumchlorid, Hexetidin, Alkylthiuroniumverbindungen, Benzylalkohol, Phenoxyethanol, Phenoxypropanole, Ethylhexylglycerin, Undecylensäure, 2-Biphenylol, Triclosan, p-Chlor-m-Xylenol und Thymol.

In einer bevorzugten Ausführungsform enthalten die erfindungsgemäßen Desinfektionsmittel eine oder mehrere flüchtige Zusatzkomponente(n) mit mikrobizider Wirkung in einer Menge von 0,001 bis 2 Gewichtsprozent, jeweils bezogen auf das gesamte Mittel.

Weiterhin können die vorzugsweise in flüssiger Form vorliegenden Desinfektionsmittel bis zu 2 Gewichtsprozent, bezogen auf das gesamte Mittel, Lösungsvermittler enthalten. Bevorzugt sind diese Lösungsvermittler ausgewählt aus der Gruppe Fettalkoholalkoxylate und/oder hydriertes Castoröl.

Weiterhin können die erfindungsgemäßen Desinfektionsmittel bis zu 2 Gewichtsprozent, bezogen auf das gesamte Mittel, Denaturierungsmittel, Farbstoffe und/oder Geruchskorrigenzien enthalten. Denaturierungsmittel, auch Vergällungsmittel genannt, sind Stoffe mit welchen Alkohole z.B. Ethanol, genussuntauglich gemacht werden. Geeignete Denaturierungsmittel sind beispielsweise Butan-2-on, Denatoniumbenzoat (Handelsname Bitrex), Diethylphthalat. Geruchskorrigenzien sind Stoffe, die den Eigengeruch von Alkoholen zumindest partiell überdecken und die nach Anwendung vorzugsweise einen Wohlgeruch auf Händen oder Oberflächen zurücklassen. Im näheren Sinne handelt es sich dabei um natürliche, naturidentische und/ oder synthetische Geruchsstoffe bzw. deren Mischungen, Parfüm und/ oder etherische Öle. Im ferneren Sinne sind aber auch geruchsneutralisierende Substanzen wie Zinkricinoleat oder Cyclodextrine möglich. Die Auswahl der vorgenannten Zusatzstoffe liegt im Ermessen des Fachmanns und kann von ihm jeweils problemlos den Wünschen und Anforderungen gemäß angepasst werden.

In einer weiteren bevorzugten Ausführungsform enthalten die erfindungsgemäßen Desinfektionsmittel zusätzlich eine Sauerstoff abspaltende Verbindung. Es hat sich herausgestellt, dass gerade in den erfindungsgemäßen Desinfektionsmitteln Persauerstoffverbindungen stabil einarbeitbar sind. Als Sauerstoff abspaltende Verbindung kommen insbesondere aliphatische Persäuren wie Peressigsäure oder Perpropionsäure und/oder aromatische Persäuren wie Perbenzoesäure oder Monoperophthalsäure sowie deren Salze, wie beispielsweise Magnesium-Monoperoxidophthalat in Frage. Weiterhin geeignet sind Glycerinmonoperacetdiacetat und/oder Glycerinmonoperacetacetat und/oder Glykolperacetacetat, die beispielsweise aus der Umsetzung von Peressigsäure mit Glycerintriacetat und/oder Glycerindiacetat und/oder Glykoldiacetat erhalten werden können. Ebenfalls gut einarbeitbar in die erfindungsgemäßen Mittel ist die Monoperoxicitronensäure. Besonders bevorzugt ist jedoch der Einsatz von Wasserstoffperoxid.

Vorzugsweise enthalten die erfindungsgemäßen Desinfektionsmittel Sauerstoff abspaltende Verbindungen in einer Menge von bis zu 1 Gewichtsprozent, bezogen auf das gesamte Mittel.

Darüber hinaus können die erfindungsgemäßen Desinfektionsmittel weitere anorganische und organische Säuren enthalten. Bevorzugt eingesetzt werden organische Säuren, wie beispielsweise Citronensäure, Milchsäure, Weinsäure, Apfelsäure, Pyroglutaminsäure, Gluconsäure und das korrespondierende Gluconodeltalacton. In einer bevorzugten Ausführungsform enthalten die erfindungsgemäßen Desinfektionsmittel zusätzlich eine der vorgenannten Säuren, insbesondere Gluconsäure und/oder das korrespondierende Gluconodeltalacton.

Die erfindungsgemäßen Desinfektionsmittel können je nach Einsatzgebiet vorzugsweise flüssig bis gelförmig formuliert werden. Dünnflüssige Mittel mit niedriger Viskosität werden bevorzugt in Sprühapplikatoren eingesetzt, da sie mit den handelsüblichen Sprühköpfen gut dosiert werden können. In einer bevorzugten Ausführungsform liegen die erfindungsgemäßen Mittel jedoch in Form eines Gels vor. Gele weisen den Vorteil auf, dass sie einfach dosiert werden können, beispielsweise aus Flüssigkeitsspendern und zudem verbesserte Lager- und Transporteigenschaften aufweisen. Durch Zugabe handelsüblicher Verdickungsmittel oder Gelbildner lassen sich die erfindungsgemäßen Desinfektionsmittel als Gele formulieren. Bevorzugt sind dabei organische Gelbildner natürlichen oder synthetischen Ursprungs, wie beispielsweise Polyacrylamid oder Polyvinylpyrrolidon sowie deren Derivate.

Weiter bevorzugte Verdickungsmittel sind ausgewählt aus der Gruppe bestehend aus Agar-Agar, Guar-Gum, Alginate, Xanthan-Gum, Gummiarabicum, Johannesbrotkernmehl, Leinsamengummen, Dextrane, Cellulose-Derivate, z.B. Methylcellulose, Hydroxyalkylcellulose und Carboxymethylcellulose, Stärkefraktionen und Derivate, wie Amylose, Amylopektin und Dextrine. Weiter bevorzugt einsetzbar sind Polyvinylalkohol sowie teilweise verseifte Polyvinylacetate und deren Derivate.

In einer bevorzugten Ausführungsform liegen die erfindungsgemäßen Desinfektionsmittel mit einer Viskosität von oberhalb 250, bevorzugt oberhalb von 500, weiter bevorzugt oberhalb von 1000, besonders bevorzugt von 2000 bis 35000, insbesondere von 3000 bis 30000, im Speziellen von 4000 bis 25000, vorteilhaft von 5000 bis 20000, beispielsweise 6000 bis 15000 mPs (Brookfield Viskosimeter LVT-II bei 4 U/min und 20 °C, Spindel 5) vor.

Die erfindungsgemäßen Desinfektionsmittel werden durch übliche, dem Fachmann geläufige Verfahren hergestellt, beispielsweise durch Mischen der Einzelkomponenten in beliebiger Reihenfolge in einer Mischvorrichtung.

Die erfindungsgemäßen Desinfektionsmittel weisen eine hervorragende viruzide Breitbandwirkung auf und sind zudem ausgesprochen hautfreundlich. Das erfindungsgemäße Desinfektionsmittel erfüllt in einer vorteilhaften Ausführungsform die Empfehlung des Robert-Koch-Instituts zur Prüfung und Deklaration der Wirksamkeit von Desinfektionsmittel gegen Viren (Bundesgesundheitsblatt-Gesundheitsforschung-Gesundheitsschutz 2004, 42, Seiten 62-66) und ist innerhalb einer Minute wirksam gegen Adenovirus Typ 5, Stamm Adenoid 75, Papovavirus [Simianvirus 40 (SV40), Stamm 777], Poliovirus (Polio-Impfstamm Typ I, Stamm LSc-2ab) und Vacciniavirus (Stamm Elstree). Darüber hinaus wurde festgestellt, dass die erfindungsgemäßen Mittel innerhalb von 15 - 30 Sekunden gegen das Bovine Viral Diarrhea Virus und das Feline Calici Virus wirksam sind. Weiterhin sind die erfindungsgemäßen Desinfektionsmittel gemäß der Prüfnorm EN 14476 innerhalb 1 Minute wirksam. Darüber hinaus wurde festgestellt, dass die erfindungsgemäßen Mittel nicht nur eine starke viruzide Breitbandwirkung aufweisen, sondern zudem bakterizid wirksam sind. Gemäß den Prüfnormen EN 1040, EN 12054, EN 1500 und prEN 12791 haben sich die erfindungsgemäßen Desinfektionsmittel als bakterizid wirkend herausgestellt.

Ebenso ist das erfindungsgemäße Desinfektionsmittel geeignet, Pilze abzutöten. Gemäß der Prüfnorm EN 1275 weisen die erfindungsgemäßen Desinfektionsmittel ebenfalls eine fungizide Wirksamkeit auf.

Ein weiterer Gegenstand der vorliegenden Erfindung ist daher die Verwendung des erfindungsgemäßen Desinfektionsmittels als Bakterizid und/oder Fungizid. Darüber hinaus ist ein weiterer Gegenstand der vorliegenden Erfindung die Verwendung des erfindungsgemäßen Desinfektionsmittels zur Abtötung von behüllten und unbehüllten Viren wie Adenovirus Typ 5, Stamm Adenoid 75), Papovavirus [Simianvirus 40 (SV40), Stamm 777], Poliovirus (Polio-Impfstamm Typ I, Stamm LSc-2ab) und Vacciniavirus (Stamm Elstree), insbesondere zur Abtötung von Bovine Viral Diarrhea Virus, des Rotavirus (Stamm WA), des Feline Calici-Virus (Surrogatvirus für NORO-Viren), des Aviären Influenza A Virus, des bovinen Coronavirus (Surrogatvirus für SARS).

In einer bevorzugten Ausgestaltung der vorliegenden Erfindung werden die Desinfektionsmittel zur hygienischen Desinfektion belebter Oberflächen, insbesondere menschlicher oder tierischer Hautpartien verwendet. Der Einsatz der erfindungsgemäßen Desinfektionsmittel zur Desinfektion belebter Oberflächen hat sich insbesondere deshalb als hervorragend erwiesen, da die Mittel eine ausgesprochen gute Hautverträglichkeit aufweisen und zudem eine starke viruzide Breitbandwirkung sowie fungizide und bakterizide Wirkung aufweisen. Die Mittel werden daher zur hygienischen Händedesinfektion, insbesondere zur chirurgischen Händedesinfektion sowie bei unbekannter Virusexposition eingesetzt. Die hervorragende Hautverträglichkeit macht die Mittel dazu geeignet, als Händedesinfektionsmittel im Dauergebrauch eingesetzt zu werden.

Vorzugsweise kommen die erfindungsgemäßen Desinfektionsmittel gewerblich in Krankenhäusern, ärztlichen Praxen, Tierarztpraxen, im Veterinärbereich (Tierhaltung), im pharmazeutischen sowie im chirurgischen Bereich zum Einsatz. Darüber hinaus eignen sich die Mittel selbstverständlich auch beim Einsatz in der Lebensmittelhygiene, beispielsweise bei der Fleischverarbeitung. Bei Bedarf kann das Mittel ferner im Privatbereich als Mittel zur Prophylaxe eingesetzt werden. Dies ist insbesondere dann sinnvoll, wenn größere Virusepedemien drohen, wie beispielsweise durch NORO-Viren, SARS, Vogelgrippe, usw.

Gerade im klinischen Bereich, der üblicherweise einer erhöhten Exposition von Viren ausgesetzt ist, hat sich der Einsatz der erfindungsgemäßen Desinfektionsmittel als besonders geeignet herausgestellt.

Besonders bevorzugt ist das erfindungsgemäße Desinfektionsmittel ein Händedesinfektionsmittel.

Gegenstand der vorliegenden Erfindung in einer weiteren Ausführungsform ist weiterhin die Verwendung des erfindungsgemäßen Desinfektionsmittels zur Desinfektion unbelebter Oberflächen, insbesondere von Türgriffen, Bettgestellen, chirurgischen Instrumenten, Krankenhaus-Einrichtungen oder von Haushaltsgegenständen.

Die erfindungsgemäßen Desinfektionsmittel werden vorzugsweise als dünnflüssige Lösungen formuliert. Für die Desinfektion größerer Flächen oder Gegenstände kann das Desinfektionsmittel in einem Behälter bereitgestellt werden, in das nachfolgend die zu desinfizierenden Gegenstände eingetaucht werden. Die Desinfektionsmittel können jedoch auch durch Besprühen oder Abwischen der zu desinfizierenden Objekte und Oberflächen desinfiziert werden. Die Desinfektionsmittel haben sich als besonders geeignet herausgestellt, da sie im Vergleich zu herkömmlichen Desinfektionsmitteln keinen Schmierfilm auf den damit behandelten desinfizierten Gegenständen hinterlassen. Schmierfilme bereiten auf Händen ein unangenehmes Hautgefühl und können im Extremfall dazu führen, dass kein sicherer Griff mehr gewährleistet ist. Bei der Applikation der erfindungsgemäßen Desinfektionsmittel auf unbelebten Oberflächen zeigen diese ebenfalls den Vorteil, dass es nicht zu einer Gleitfilmbildung auf den behandelten Oberflächen kommt.

Das Desinfektionsverfahren kann sehr einfach ausgeführt werden. Gemäß eines weiteren bevorzugten Gegenstands der vorliegenden Erfindung umfasst das Desinfektionsverfahren die folgenden Schritte:
a) Applizieren des erfindungsgemäßen Desinfektionsmittels auf eine kontaminierte Oberfläche und
b) Einwirkenlassen des Desinfektionsmittels über einen Zeitraum der ausreicht, Viren, Pilze und/oder Bakterien abzutöten.

Das erfindungsgemäße Desinfektionsverfahren ist für den in Frage kommenden Personenkreis, beispielsweise Klinikpersonal, einfach handhabbar. In einer bevorzugten Ausführungsform des erfindungsgemäßen Verfahrens erfolgt das Applizieren durch Aufsprühen und/oder Einreiben des Desinfektionsmittels auf die Oberfläche oder durch Eintauchen der Oberfläche in das Desinfektionsmittel. In einer weiteren bevorzugten Ausführungsform handelt es sich bei der Oberfläche um menschliche Haut, insbesondere um Hände. Gerade im klinischen und chirurgischen Bereich hat sich der Einsatz von Erzeugnissen, die das Desinfektionsmittel enthalten, besonders bewährt. Geeignete Erzeugnisse können beispielsweise Sprühapplikatoren oder Flüssigkeitsspender sein, die beispielsweise in einem Hygieneraum angebracht sind, in dem die Desinfektion vorgenommen werden soll. Sprühapplikatoren und Flüssigkeitsspender, die in der Nähe von Waschbecken angebracht sind, sind dabei von besonderer Bedeutung. So kann beispielsweise zunächst eine grobe Vorreinigung der Hautpartien von Verunreinigungen mittels üblicher Reinigungsmittel vorgenommen werden und nachfolgend ein Desinfektionsschritt mit dem erfindungsgemäßen Desinfektionsmittel durchgeführt werden, bei dem beispielsweise die Hände mit einem Sprühapplikator eingesprüht werden oder bei dem die Hände und/oder Hautpartien mit dem Desinfektionsmittel eingerieben werden.

Neben Sprühapplikatoren haben sich bevorzugt auch solche Erzeugnisse herausgestellt, bei denen das erfindungsgemäße Desinfektionsmittel auf einem festen Träger vorliegt, der mit dem Desinfektionsmittel getränkt oder behandelt wurde. Vorzugsweise handelt es sich bei den erfindungsgemäßen Erzeugnissen um imprägnierte oder getränkte textile Flächengebilde oder imprägnierte oder getränkte Papiere. Beispielsweise können Baumwoll- bzw. Cellulosetex-tilien oder auch Polypropylenvlies mit dem erfindungsgemäßen Desinfektionsmittel getränkt und/oder imprägniert werden und anschließend in einer Dosiervorrichtung für Tücher dargereicht werden.

Die vorliegende Erfindung wird durch die nachfolgenden Beispiele näher beschrieben ohne jedoch darauf beschränkt zu sein.

### Beispiele:

Um das Wesen der Erfindung zu demonstrieren, wurden Rezepturen nachfolgender Zusammensetzungen angesetzt. Viruzidie-Prüfungen, die gemäss der RKI-Viruzidie-Empfehlung unter den Standardprüfbedingungen durchgeführt wurden, beziehen sich auf die Prüfviren Adenovirus Typ 5, Stamm Adenoid 75), Papovavirus [Simianvirus 40 (SV40), Stamm 777], Poliovirus (Polio-Impfstamm Typ I, Stamm LSc-2ab), Vacciniavirus (Stamm Elstree) sowie auf den Poliovirus Typ 1 Stamm Mahoney. Aus Gründen der Lesbarkeit werden die Bezeichnungen mit Polio, Adeno, Vaccinia, Papova abgekürzt.

### Beispiel 1

**Tabelle 1: Rezeptur eines handelsüblichen Produkts**

| Rezepturbestandteil | Rezeptur | Viruswirksamkeit |
|---|---|---|
| Ethanol | 52,4 g | Nicht ausreichend gegen Polio wirksam, damit nicht viruzid gemäss der RKI-Viruzidie-Empfehlung. |
| Propan-1-ol | 21,0 g | |
| Dexpanthenol | 0,5 g | |
| PEG 6 Caprylic Capric Glycerides | 1,0 g | |
| Diisopropyladipat, Allantoin, Bisabolol, Vergällungsmittel, Wasser | ad. 100 g | |

### Beispiel 2

**Tabelle 2: Vergleichsrezeptur mit einer kurzkettigen organischen Säure**

| Rezepturbestandteil | Rezeptur | Viruswirksamkeit |
|---|---|---|
| Ethanol | 52,4 g | Nicht ausreichend gegen Polio wirksam, damit |
| Propan-1-ol | 21,0 g | |
| Citronensäure | 0,5 g | nicht viruzid gemäss der RKI Viruzidie-Empfehlung. |
| Polyethylenglykol 4000 | 1,0 g | |
| Glycerin, Octyldodecanol, Butan-2-on, Wasser | ad. 100 g | |

### Beispiel 3

**Tabelle 3: Vergleichsrezeptur mit Diolen**

| Rezepturbestandteil | Rezeptur | Viruswirksamkeit |
|---|---|---|
| Ethanol | 52,4 g | Unter den Standardprüf-bedingungen innerhalb von 1 Minute nicht ausreichend gegen Polio, Papova und Adeno wirksam. |
| Propan-1-ol | 21,0 g | |
| Citronensäure | 0,5 g | |
| Propan-1,2-diol | 5,0 g | |
| Butan-1,3-diol | 5,0 g | |
| Butan-2-on, Wasser | ad. 100 g | |

### Beispiel 4

**Tabelle 4: Vergleichsversuch ohne den Einsatz von Alkylenglycol**

| Rezepturbestandteil | Rezeptur | Viruswirksamkeit |
|---|---|---|
| Ethanol | 54,1 g | Unter den Standardprüfbedingungen innerhalb von 1 Minute nicht ausreichend gegen Papova und Adeno wirksam. |
| Propan-1-ol | 10,0 g | |
| Phosphorsäure | 0,7 g | |
| Propan-1,2-diol | 5,9 g | |
| Butan-1,3-diol | 5,7 g | |
| Hilfsstoffe, Wasser | ad. 100 g | |

### Beispiel 5

**Tabelle 5: Desinfektionsmittel zur Händedesinfektion**

| Rezepturbestandteil | Rezeptur | Viruswirksamkeit |
|---|---|---|
| Ethanol | 52,4 g | Das Produkt ist unter Standardprüfbedingungen innerhalb von 1 Minute wirksam gegen Polio, Adeno, Vaccinia, Papova und damit viruzid gemäss der RKI Viruzidie-Empfehlung. |
| Propan-1-ol | 21,0 g | |
| Phosphorsäure | 0,7 g | |
| Polyethylenglykol 4000 | 1,0 g | |
| Glycerin, Octyldodecanol, Butan-2-on, Wasser | ad. 100 g | |

Gegen das Feline Calici-Virus (Surrogatvirus für das NORO-Virus), das Bovine Viral Diarrhea Virus (Surrogat-Virus für das Hepatitis C-Virus), das Rotavirus (Stamm WA), das bovine Corona-Virus (Surrogat-Virus für SARS) und das Aviäre Influenza A Virus H7N1 (Surrogat-Virus für das Vogelgrippe-Virus H5N1) ist das Mittel gemäß Beispiel 5 innerhalb von 15 Sekunden wirksam. Der Flammpunkt der Mischung beträgt 21°C.

Das Händedesinfektionsmittel wurde gegen Polio, Adeno, Vaccinia und Papova unverdünnt bei 20°C untersucht. Bedingt durch die Zugabe der Virussuspension und der organischen Belastungen resultierte eine 80,0%ige Prüfkonzentration (Standardansatz). Die gewählten Einwirkzeiten beliefen sich auf 0,5, 1,0 und 2,0 Minuten.

Das geprüfte Händedesinfektionsmittel war unverdünnt in der Lage, in allen Ansätzen nach einer Minute Einwirkzeit eine Titerreduktion von mindestens 4 log₁₀-Stufen zu erreichen. Bei den Untersuchungen gegen Polio konnte nach 2 Minuten Einwirkzeit dann kein Virus mehr detektiert werden. Die maximal messbaren Titerreduktionen betrugen 5 log₁₀-Stufen.

Die genannten Titerreduktionen bzw. Reduktionsfaktoren bedeuten eine Inaktivierung von 99,99% bzw. 99,999% und damit eine ausreichende Virus-Wirksamkeit. Bekanntlich wird in der Richtlinie des BGA und der DW immer dann von einer Virus-Wirksamkeit ausgegangen, wenn eine Titerreduktion von 4 log₁₀-Stufen (Inaktivierung 99,99%) darstellbar ist.

Zusätzlich ist das Händedesinfektionsmittel als 90,0%ige Lösung ohne Belastung und in Gegenwart von BSA (BGA/DW-Belastung) und unter hoher Belastung ("dirty conditions") nach der EN 14476:2005 überprüft worden. Nach 30 Sekunden ergaben sich auch hier Reduktionsfaktoren von mindestens 4 log₁₀-Stufen.

Die als Kontrolle mitgeführte Formaldehyd-Lösung reduzierte den Poliovirus-Titer nach 15 bzw. 30 Minuten Einwirkzeit um 1,25 bzw. 2,13 log₁₀-Stufen. Nach 60 bzw. 120 Minuten betrugen die Reduktionsfaktoren 2,75 bzw. 3,13.

### Beispiel 6

**Tabelle 6: Erfindungsgemäßes Desinfektionsmittel zur Händedesinfektion**

| Rezepturbestandteil | Rezeptur |
|---|---|
| Ethanol | 52,4 g |
| Propan-1-ol | 21,0 g |
| Phosphorsäure | 0,7 g |
| Natriumdihydrogenphosphat | 0,2 g |
| Polyethylenglykol 4000 | 1,0 g |
| Glycerin, Octyldodecanol, Butan-2-on, Wasser | ad. 100 g |

### Beispiel 7

**Tabelle 7: Erfindungsgemäßes Desinfektionsmittel zur Händedesinfektion**

| Rezepturbestandteil | Rezeptur |
|---|---|
| Ethanol | 52,4 g |
| Propan-1-ol | 21,0 g |
| Phosphorsäure | 0,4 g |
| Natriumdihydrogenphosphat | 0,3 g |
| Polyethylenglykol 4000 | 1,0 g |
| Glycerin, Octyldodecanol, Butan-2-on, Wasser | ad. 100 g |

### Beispiel 8

**Tabelle 8: Desinfektionsmittel zur Händedesinfektion**

| Rezepturbestandteil | Rezeptur |
|---|---|
| Ethanol | 52,4 g |
| Propan-1-ol | 21,0 g |
| Phosphorsäure | 0,8 g |
| Polyethylenglykol 4000 | 1,0 g |
| Wasserstoffperoxid | 0.3 g |
| Glycerin, Octyldodecanol, Butan-2-on, Wasser | ad. 100 g |

### Beispiel 9

**Tabelle 9**

| | Vergleichsbeispiel 1 (gem. Example 9 der US2004/0146479) mit Ethanol | Vergleichsbeispiel 2 (gem. Example 9 der US2004/0146479) mit Ethanol/ Propan-1-ol |
|---|---|---|
| Gereinigtes Wasser | ad 100 ml | ad 100 g |
| Ethanol | 55,23 g | 55,70 g |
| Butan-2-on | | 1,00 g |
| Propan-1-ol | | 20,00 g |
| Triclosan | 0,50 g | 0,50 g |
| Propylenglycol | 0,50 g | 0,50 g |
| Benzyl Alcohol | 0,35 g | 0,35 g |
| PEG 75 Lanolin (50%) | 0,50 g | 0,50 g |
| PEG 400 | 6,00 g | 6,00 g |
| PEG 4000 | 2,00 g | 2,00 g |
| Phosphorsäure (85 %) | 0,12 g | 0,12 g |
| Triethanolamin | 0,10 g | 0,10 g |
| Kaliumiodid | 0,20 g | < 0,20 g*) |
| *) < 0,20 g da keine vollständige Lösung des KI | | |

Anmerkung: Povidon-lod wurde in den Vergleichsbeispielen 1 und 2 nicht verwendet, da dies selbst eine viruzide Wirksamkeit aufweist und somit die zu beobachtenden Effekte verfälschen würde.

Bedingt durch die Zugabe der Virussuspension und der interferierenden Substanz (FKS) resultierte eine 80,0%ige Prüfkonzentration. Die Einwirkzeiten betrugen 30, 60 und 120 Sekunden.

Die geprüften Vergleichsbeispiele 1 und 2 verursachten bei 20°C (+/- 1°C) innerhalb der geprüften Einwirkzeiten keine Inaktivierung des Poliovirus Typ 1 Stamm LSc-2ab. So sind sogar auch Virustiter ermittelt worden, die bedingt durch die Schwankungsbreite der Messungen oberhalb der entsprechenden Viruskontrollen lagen. Auch nach einer Einwirkzeit von zwei Minuten wurde bei den Vergleichsbeispielen 1 und 2 keinerlei Titerreduktion erreicht.

Die Vergleichsbeispiele 1 und 2 müssen deshalb als unwirksam gegen Polioviren angesehen werden.

Die erfindungsgemäßen Desinfektionsmittel erwiesen sich als wirksam gegen das Hepatitis A Virus sowie gegen Parvoviren und erfüllten die Vorgaben der Prüfnorm EN 14476. Die Produkte erwiesen sich zudem als wirksam zur hygienischen Händedesinfektion gemäss EN 1500 innerhalb von 15 Sekunden sowie zur chirurgischen Händedesinfektion gemäss prEN 12791 innerhalb von 1,5 Minuten und wiesen eine fungizide Wirksamkeit gemäss EN 1275 auf. In einem mehrwöchigen Anwendungstest wurde die Akzeptanz der erfindungsgemäßen Rezepturen geprüft und mit positivem Ergebnis beurteilt. Das Produkt erzielte eine gute Verträglichkeit, auch bei häufiger Anwendung, bei verlängerter Einwirkzeit und beim Tragen von Handschuhen. Das Produkt erwies sich unter ICH-Bedingungen als lagerstabil. Die Aufgabe, ein viruswirksames Händedesinfektionsmittel mit Breitbandwirkung für den Routinegebrauch zu entwickeln, wurde damit zufriedenstellend gelöst.

## Patentansprüche

1. Viruzides Desinfektionsmittel mit Breitbandwirkung umfassend
a) eine oder mehrere saure Phosphorverbindung(en), die ausgewählt ist/sind aus der Gruppe der Phosphorsäure, Diphosphorsäure, Triphosphorsäure, Polyphosphorsäure der allgemeinen Formel Hₙ₊₂PₙO₃ₙ₊₁ mit n gleich eine ganze Zahl von 1 bis 17, cyclo-Tri- und Tetrametaphosphorsäure, Polymet-phosphorsäure, Peroxomonophosphorsäure, Peroxodiphosphorsäure, Hypophosphorsäure, Diphosphor(III, IV)-säure, sowie der Salze dieser Säuren, in einer Menge von 0,2 bis 1,5 Gew.-%, bezogen auf das Mittel, mit der Maßgabe, dass die sauren Phosphorverbindungen Phosphorsäure und ein oder mehrere Alkalimetallsalze der Phosphorsäure umfasst und die Phosphorsäure und die ein oder mehreren Alkalimetallsalze der Phosphorsäure in einer Menge von 0,2 bis 1,5 Gew.-%, bezogen auf das Mittel, vorliegen,
b) als alkoholische Komponente Ethanol und/oder Propan-1-ol und/oder Propan-2-ol, vorzugsweise Mischungen aus Ethanol und Propon-1-ol, in einer Menge von 30 bis 80 Gew.-%, bezogen auf das gesamte Mittel, und
c) einen oder mehrere Polyalkylenglykol(e), der allgemeinen Formel HO-[R-O]ₙ-H, in der R für (CH₂)₂, CH₂CH(CH₃), CH₂CH(CH₂CH₃) oder für (CH₂)₄ und n für Werte von 2 bis 200 steht, enthält.

2. Desinfektionsmittel nach Anspruch 1, **dadurch gekennzeichnet, dass** es Phosphorsäure und/oder Natriumdihydrogenphosphat und/oder Kaliumdihydrogenphosphat, besonders bevorzugt Mischungen aus Phosphorsäure mit Natriumdihydrogenphosphat und/oder Kaliumdihydrogenphosphat enthält.

3. Desinfektionsmittel nach einem oder mehreren der Ansprüche 1 oder 2, **dadurch gekennzeichnet, dass** es die eine oder mehrere saure Phosphorverbindung(en) in einer Menge von 0,2 bis 1,0 Gew.-%, vorzugsweise 0,2 bis 0,8 Gew.-%, jeweils bezogen auf das Mittel, enthält.

4. Desinfektionsmittel nach einem oder mehreren der Ansprüche 1 bis 3, **dadurch gekennzeichnet, dass** das Polyalkylenglykol ein Polypropylenglykol und/oder ein Ethylenoxid/Propylenoxid-Blockcopolymer und/oder vorzugsweise ein Polyethylenglykol ist.

5. Desinfektionsmittel nach einem oder mehreren der Ansprüche 1 bis 4, **dadurch gekennzeichnet, dass** die mittlere relative Molmasse des/der Polyalkylenglykol(e) 400 bis 10000, vorzugsweise 1000 bis 8000 und insbesondere 2000 bis 6000 beträgt.

6. Desinfektionsmittel nach einem oder mehreren der Ansprüche 1 bis 5, **dadurch gekennzeichnet, dass** es die alkoholische Komponente in einer Menge von 45 bis 80 Gew.-% und insbesondere von 60 bis 75 Gew.-%, jeweils bezogen auf das gesamte Mittel, enthält.

7. Desinfektionsmittel nach einem oder mehreren der Ansprüche 1 bis 6, **dadurch gekennzeichnet, dass** es einen Flammpunkt (gemessen nach DIN 51755) von wenigstens 21 °C aufweist.

8. Desinfektionsmittel nach einem oder mehreren der Ansprüche 1 bis 7, **dadurch gekennzeichnet, dass** es zusätzlich 0,01- 5 Gew.- %, vorzugsweise 0,1 - 2 Gew.-%, jeweils bezogen auf das gesamte Mittel, einer oder mehrerer Hautpflegekomponente(n) enthält, die ausgewählt ist/sind aus der Gruppe bestehend aus Glycerin, Propan-1,2-diol, Butan-1,3-diol, Sorbitol, Dexpanthenol, Allantoin, Bisabolol, Tocopherylacetat, Octyldodecanol, Dodecanol, Tetradecanol, Hexadecanol, Octadecanol, Lanolinalkohol, Cetearylalkohol, Cyclomethicone, Dimethicone, Isopropylmyristat, Isopropylpalmitat, Cetearylethylhexanoat, Octylstearat, Octyloctanoat, Ethylhexansäureethylester, Jojobaöl, Sanddornöl, Wollwachs, Paraffinöl, Vaseline, Heptamethylnonan/Isohexadecan, Cholesterol, Partialglyceriden und -triglyceriden.

9. Desinfektionsmittel nach einem oder mehreren der Ansprüche 1 bis 8, **dadurch gekennzeichnet, dass** es 0,001 - 2 Gew. %, jeweils bezogen auf das gesamte Mittel, einer oder mehrerer nicht flüchtigen/r Zusatzkomponente(n) mit mikrobizider Wirkung enthalten, ausgewählt aus der Gruppe Senzalkoniumchlorid, Didecyldimethylammoniumchlorid, Mecetroniumetilsulfat, Octenidin, Polyhexamethylenbiguanid, Chlorhexidingluconat, Chlorhexidinacetat, Cetrimid, Cetylpyridiniumchlorid, Hexetidin, Alkylthiuroniumverbindungen, Benzylalkohol, Phenoxyethanol, Phenoxypropanole, Ethylhexylglycerin, Undecylensäure, 2-Biphenylol, Triclosan, p-Chlor-m-Xylenol und Thymol.

10. Desinfektionsmittel nach einem oder mehreren der Ansprüche 1 bis 9, **dadurch gekennzeichnet, dass** es bis zu 2 Gew. %, bezogen auf das gesamte Mittel, Lösungsvermittler ausgewählt aus der Gruppe hydriertes Castor Öl und/oder Fettalkoholalkoxylate enthält.

11. Desinfektionsmittel nach einem oder mehreren der Ansprüche 1 bis 10, **dadurch gekennzeichnet, dass** es zusätzlich eine sauerstoffabspaltende Verbindung, vorzugsweise Wasserstoffperoxid enthält, besonders bevorzugt in einer Menge bis zu 1,0 Gew.-%, bezogen auf das gesamte Mittel.

12. Desinfektionsmittel nach einem oder mehreren der Ansprüche 1 bis 11, **dadurch gekennzeichnet, dass** es zusätzlich Rückfetter, ausgewählt aus der Gruppe bestehend aus Glycerintri-, Glycerindi- und Glycerinmonooleat, Glycerylcaprylat, Glycerylcaprat, Polyglyceryl-2-caprat sowie langkettige lineare oder verzweigte ein- oder mehrwertige Fettalkohole, insbesondere Octyldodecanol, Isopropylmyristat und Cetearyloctanoat, enthält.

13. Desinfektionsmittel nach einem oder mehreren der Ansprüche 1 bis 12, **dadurch gekennzeichnet, dass** es als Gel vorliegt.

14. Desinfektionsmittel nach einem oder mehreren der Ansprüche 1 bis 13, **dadurch gekennzeichnet, dass** es die Empfehlung des Robert Koch Institutes zur Prüfung und Deklaration der Wirksamkeit von Desinfektionsmitteln gegen Viren erfüllt und innerhalb einer Minute wirksam gegen Adenovirus Typ 5, Stamm Adenoid 75), Papovavirus [Simianvirus 40 (SV40), Stamm 777], Poliovirus (Polio-Impfstamm Typ I, Stamm LSc-2ab) und Vacciniavirus (Stamm Elstree) sind.

15. Verwendung eines Desinfektionsmittels gemäß einem oder mehreren der Ansprüche 1 bis 14 zur hygienischen Desinfektion belebter Oberflächen, insbesondere menschlicher oder tierischer Hautpartien.

16. Verwendung nach Anspruch 15, **dadurch gekennzeichnet, dass** das Mittel zur hygienischen Händedesinfektion, zur chirurgischen Händedesinfektion und bei unbekannter Virusexposition eingesetzt wird.

17. Verwendung nach einem der Ansprüche 15 oder 16, **dadurch gekennzeichnet, dass** das Mittel zur Händedesinfektion im Dauergebrauch eingesetzt wird.

18. Verwendung eines Desinfektionsmittels nach einem oder mehreren der Ansprüche 1 bis 14 zur Desinfektion unbelebter Oberflächen, insbesondere von Türgriffen, Bettgestellen, chirurgischen Instrumenten, Krankenhauseinrichtungen oder von Haushaltsgegenständen.

19. Verwendung eines Desinfektionsmittels gemäß einem oder mehreren der Ansprüche 1 bis 14 zur Abtötung von Adenovirus Typ 5, (Stamm Adenoid 75), Papovavirus [Simianvirus 40 (SV40), Stamm 777], Poliovirus (Polio-Impfstamm Typ I, Stamm LSc-2ab), Vacciniavirus (Stamm Elstree), Bovine Viral Diarrhea Virus, und Feline Calici-Viren.

20. Verwendung eines Desinfektionsmittels gemäß einem oder mehreren der Ansprüche 1 bis 14 als Bakterizid und/oder Fungizid.

21. Desinfektionsverfahren umfassend die folgenden Schritte,
a. Applizieren eines Desinfektionsmittels nach einem oder mehreren der Ansprüche 1 bis 14 auf eine kontaminierte Oberfläche und
b. Einwirkenlassen des Desinfektionsmittel über einen Zeitraum der ausreicht Viren, Pilze und/oder Bakterien abzutöten.

22. Verfahren nach Anspruch 21, **dadurch gekennzeichnet, dass** das Applizieren durch Aufsprühen und/oder Einreiben des Desinfektionsmittels auf die Oberfläche oder durch Eintauchen der Oberfläche in das Desinfektionsmittel erfolgt.

23. Verfahren nach Anspruch 21 oder 22, **dadurch gekennzeichnet, dass** es sich bei der Oberfläche um menschliche Haut, insbesondere Hände handelt.

24. Erzeugnis enthaltend ein Desinfektionsmittel gemäß einem oder mehreren der Ansprüche 1 bis 14.

25. Erzeugnis gemäß Anspruch 24, **dadurch gekennzeichnet, dass** es sich um einen Sprühapplikator oder einen getränkten oder mit dem Desinfektionsmittel behandelten festen Träger handelt, vorzugsweise um imprägnierte oder getränkte textile Flächengebilde, getränktes oder imprägniertes Kunststoff-Vlies oder imprägnierte oder getränkte Papiere.

## Claims

1. A virucidal disinfectant with broad range activity, comprising:
a) one or more acidic phosphorus compounds selected from the group consisting of phosphoric acid, diphosphoric acid, triphosphoric acid, polyphosphoric acid of general formula Hₙ₊₂PₙO₃ₙ₊₁, with n being an integer of from 1 to 17, cyclotrimetaphosphoric acid, cyclotetra-metaphosphoric acid, polymetaphosphoric acid, peroxomonophos-phoric acid, peroxodiphosphoric acid, hypophosphoric acid, diphosphoric(III,IV) acid, and salts of these acids, in an amount of from 0.2 to 1.5% by weight, based on said disinfectant, with the proviso that said acidic phosphorus compounds include phosphoric acid and one or more alkali metal salts of phosphoric acid, and said phosphoric acid and one or more alkali metal salts of phosphoric acid are present in an amount of from 0.2 to 1.5% by weight, based on said disinfectant;
b) as the alcoholic component: ethanol and/or propan-1-ol and/or propan-2-ol, preferably mixtures of ethanol and propan-1-ol, in an amount of from 30 to 80% by weight, based on the entire disinfectant; and
c) one or more polyalkylene glycols of general formula HO-[R-O]ₙ-H, wherein R represents (CH₂)₂, CH₂CH(CH₃), CH₂CH(CH₂CH₃) or (CH₂)₄, and n represents values of from 2 to 200.

2. The disinfectant according to claim 1, **characterized by** containing phosphoric acid and/or sodium dihydrogenphosphate and/or potassium dihydrogenphosphate, more preferably mixtures of phosphoric acid with sodium dihydrogenphosphate and/or potassium dihydrogenphosphate.

3. The disinfectant according to one or more of claims 1 or 2, **characterized by** containing said one or more acidic phosphorus compounds in an amount of from 0.2 to 1.0% by weight, preferably from 0.2 to 0.8% by weight, respectively based on said disinfectant.

4. The disinfectant according to one or more of claims 1 to 3, **characterized in that** said polyalkylene glycol is a polypropylene glycol and/or an ethylene oxide/propylene oxide block copolymer and/or preferably a polyethylene glycol.

5. The disinfectant according to one or more of claims 1 to 4, **characterized in that** the average relative molecular mass of said polyalkylene glycol(s) is from 400 to 10,000, preferably from 1000 to 8000, and especially from 2000 to 6000.

6. The disinfectant according to one or more of claims 1 to 5, **characterized by** containing said alcoholic component in an amount of from 45 to 80% by weight, especially from 60 to 75% by weight, respectively based on the entire disinfectant.

7. The disinfectant according to one or more of claims 1 to 6, **characterized by** having a flash point (measured according to DIN 51755) of at least 21 °C.

8. The disinfectant according to one or more of claims 1 to 7, **characterized by** additionally containing from 0.01 to 5% by weight, preferably from 0.1 to 2% by weight, respectively based on the entire disinfectant, of one or more skin care components selected from the group consisting of glycerol, propan-1,2-diol, butane-1,3-diol, sorbitol, dexpanthenol, allantoin, bisabolol, tocopheryl acetate, octyldodecanol, dodecanol, tetradecanol, hexadecanol, octadecanol, lanolin alcohol, cetearyl alcohol, cyclomethicone, dimethicone, isopropyl myristate, isopropyl palmitate, cetearyl ethylhexanoate, octyl stearate, octyl octanoate, ethylhexanoic acid ethyl ester, jojoba oil, sea buckthorn oil, lanolin, paraffin oil, vaseline, heptamethylnonane/isohexadecane, cholesterol, partial glycerides and triglycerides.

9. The disinfectant according to one or more of claims 1 to 8, **characterized by** containing from 0.001 to 2% by weight, respectively based on the entire disinfectant, of one or more non-volatile additional components having microbicidal activity, selected from the group consisting of benzalkonium chloride, didecyldimethylammonium chloride, mecetronium etilsulfate, octenidine, polyhexamethylene biguanide, chlorohexidine gluconate, chlorohexidine acetate, cetrimide, cetylpyridinium chloride, hexetidine, alkylthiuronium compounds, benzyl alcohol, phenoxyethanol, phenoxypropanols, ethylhexylglycerol, undecylenic acid, 2-biphenylol, triclosan, p-chloro-m-xylenol and thymol.

10. The disinfectant according to one or more of claims 1 to 9, **characterized by** containing up to 2% by weight, based on the entire disinfectant, of solubilizers selected from the group consisting of hydrogenated castor oil and/or fatty alcohol alkoxylates.

11. The disinfectant according to one or more of claims 1 to 10, **characterized by** additionally containing an oxygen-releasing compound, preferably hydrogen peroxide, more preferably in an amount of up to 1.0% by weight, based on the entire disinfectant.

12. The disinfectant according to one or more of claims 1 to 11, **characterized by** additionally containing refatting agents selected from the group consisting of glycerol trioleate, glycerol dioleate and glycerol monooleate, glyceryl caprylate, glyceryl caprate, polyglyceryl 2-caprate and long-chained, linear or branched, mono- or polyhydric fatty alcohols, especially octyldodecanol, isopropyl myristate and cetearyl octanoate.

13. The disinfectant according to one or more of claims 1 to 12, **characterized by** being in the form of a gel.

14. The disinfectant according to one or more of claims 1 to 13, **characterized by** meeting the recommendation by the Robert Koch Institut for the testing and declaration of the effectiveness of disinfectants against viruses, being effective within one minute against adenovirus type 5 (adenoid 75 strain), papovavirus [simian virus 40 (SV40), strain 777], poliovirus (polio vaccination strain type I, strain LSc-2ab) and vaccinia virus (Elstree strain).

15. Use of a disinfectant according to one or more of claims 1 to 14 for the hygienic disinfection of animate surfaces, especially human or animal skin portions.

16. The use according to claim 15, **characterized in that** said disinfectant is employed for hygienic hand disinfection, surgical hand disinfection and upon exposure to unknown viruses.

17. The use according to either of claims 15 or 16, **characterized in that** said disinfectant is employed for hand disinfection in continuous use.

18. Use of a disinfectant according to one or more of claims 1 to 14 for the disinfection of inanimate surfaces, especially door handles, bedsteads, surgical instruments, hospital equipment or household articles.

19. Use of a disinfectant according to one or more of claims 1 to 14 for the killing of adenovirus type 5 (adenoid 75 strain), papovavirus [simian virus 40 (SV40), strain 777], poliovirus (polio vaccination strain type I, strain LSc-2ab), vaccinia virus (Elstree strain), bovine viral diarrhea virus, and feline caliciviruses.

20. Use of a disinfectant according to one or more of claims 1 to 14 as a bactericidal and/or fungicidal agent.

21. A disinfection method, comprising the following steps:
a. applying a disinfectant according to one or more of claims 1 to 14 to a contaminated surface; and
b. allowing the disinfectant to act for a period of time sufficient to kill off viruses, fungi and/or bacteria.

22. The method according to claim 21, **characterized in that** said applying is effected by spraying and/or rubbing the disinfectant onto the surface or by immersing the surface into the disinfectant.

23. The method according to claim 21 or 22, **characterized in that** said surface is human skin, especially of the hands.

24. A product containing a disinfectant according to one or more of claims 1 to 14.

25. The product according to claim 24, **characterized by** being a sprayer or a solid support soaked or treated with the disinfectant, preferably impregnated or soaked textile sheets, soaked or impregnated non-woven plastic materials or impregnated or soaked papers.

## Revendications

1. Désinfectant virucide à large spectre, comprenant:
a) un ou plusieurs composés de phosphore acides choisis dans le groupe consistant en l'acide phosphorique, l'acide diphosphorique, l'acide triphosphorique, l'acide polyphosphorique avec la formule générale Hₙ₊₂PₙO₃ₙ₊₁, avec n égal à un nombre entier de 1 à 17, les acides trimétaphosphorique et tétramétaphosphorique, l'acide polymétaphosphorique, l'acide peroxomonophosphorique, l'acide peroxodiphosphorique, l'acide hypophosphorique, l'acide diphosphorique(III,IV), et les sels de ces acides, en une quantité de 0,2 à 1,5 % en poids, par rapport au désinfectant, à condition que lesdits composés de phosphore acides comprennent l'acide phosphorique et/ou un ou plusieurs sels de métaux alcalins de l'acide phosphorique en une quantité de 0,2 à 1,5 % en poids, par rapport au désinfectant,
b) comme composant alcoolique, éthanol et/ou propan-1-ol et/ou propan-2-ol, de préférence des mélanges d'éthanol et de propan-1-ol, en une quantité de 30 à 80 % en poids, par rapport au désinfectant entier, et
c) un ou plusieurs polyalkylène glycols avec la formule générale HO-[R-O]ₙ-H, où R représente (CH₂)₂, CH₂CH(CH₃), CH₂CH(CH₂CH₃) ou (CH₂)₄ et n représente des valeurs de 2 à 200.

2. Désinfectant selon la revendication 1, **caractérisé en ce qu'**il contient de l'acide phosphorique et/ou le dihydrogénophosphate de sodium et/ou le dihydrogénophosphate de potassium, de préférence encore des mélanges d'acide phosphorique et de dihydrogénophosphate de sodium et/ou de dihydrogénophosphate de potassium.

3. Désinfectant selon l'une ou plusieurs des revendications 1 ou 2, **caractérisé en ce qu'**il contient ledit un ou lesdits plusieurs composés de phosphore acides en une quantité de 0,2 à 1,0 % en poids, de préférence de 0,2 à 0,8 % en poids, respectivement par rapport au désinfectant.

4. Désinfectant selon l'une ou plusieurs des revendications 1 à 3, **caractérisé en ce que** le polyalkylène glycol est un polypropylène glycol et/ou un copolymère séquencé d'oxyde d'éthylène/d'oxyde de propylène et/ou de préférence un polyéthylène glycol.

5. Désinfectant selon l'une ou plusieurs des revendications 1 à 4, **caractérisé en ce que** le poids moléculaire moyen relatif du ou des polyalkylène glycols est de 400 à 10000, de préférence de 1000 à 8000, plus particulièrement de 2000 à 6000.

6. Désinfectant selon l'une ou plusieurs des revendications 1 à 5, **caractérisé en ce qu'**il contient ledit composant alcoolique en une quantité de 45 à 80 % en poids, plus particulièrement de 60 à 75 % en poids, respectivement par rapport au désinfectant entier.

7. Désinfectant selon l'une ou plusieurs des revendications 1 à 6, **caractérisé en ce qu'**il a un point d'éclair (mesuré selon DIN 51755) d'au moins 21 °C.

8. Désinfectant selon l'une ou plusieurs des revendications 1 à 7, **caractérisé en ce qu'**il contient en outre de 0,01 à 5 % en poids, de préférence de 0,1 à 2 % en poids, respectivement par rapport au désinfectant entier, d'un ou plusieurs composants de soins de la peau choisis dans le groupe consistant en la glycérine, le propan-1,2-diol, le butan-1,3-diol, le sorbitol, le dexpanthénol, l'allantoïne, le bisabolol, l'acétate de tocophéryle, l'octyldodécanol, le dodécanol, le tétradécanol, l'hexadécanol, l'octadécanol, l'alcool lanoline, l'alcool cétéarylique, la cyclométhicone, la diméthicone, le myristate d'isopropyle, le palmitate d'isopropyle, l'éthylhexanoate de cétéaryle, le stéarate d'octyle, l'octanoate d'octyle, l'éthylhexanoate d'éthyle, l'huile de jojoba, l'huile d'argousier, la cire de laine, l'huile de paraffine, la vaseline, l'heptaméthylnonane/isohexadécane, le cholestérol, des glycérides et triglycérides partiels.

9. Désinfectant selon l'une ou plusieurs des revendications 1 à 8, **caractérisé en ce qu'**il contient en outre de 0,001 à 2 % en poids, respectivement par rapport au désinfectant entier, d'un ou plusieurs composants additionnels non volatils ayant une activité microbicide, choisis dans le groupe consistant en le chlorure de benzalkonium, le chlorure de didécyldiméthylammonium, l'éthylsulfate de mécétronium, l'octénidine, le polyhexaméthylènebiguanide, le gluconate de chlorhexidine, l'acétate de chlorhexidine, le cétrimide, le chlorure de cétylpyridinium, l'hexétidine, des composés alkylthiuronium, l'alcool benzylique, le phénoxyéthanol, les phénoxypropanols, l'éthylhexylglycérol, l'acide undécylénique, le 2-biphénylol, le triclosan, le p-chloro-m-xylénol et le thymol.

10. Désinfectant selon l'une ou plusieurs des revendications 1 à 9, **caractérisé en ce qu'**il contient jusqu'à 2 % en poids, par rapport au désinfectant entier, d'un solubilisant choisi dans le groupe consistant en l'huile de ricin hydrogénée et/ou des alkoxylates d'alcools gras.

11. Désinfectant selon l'une ou plusieurs des revendications 1 à 10, **caractérisé en ce qu'**il contient en outre un composé libérant de l'oxygène, de préférence du peroxyde d'hydrogène, de préférence encore en une quantité jusqu'à 1,0 % en poids, par rapport au désinfectant entier.

12. Désinfectant selon l'une ou plusieurs des revendications 1 à 11, **caractérisé en ce qu'**il contient en outre des agents de regraissage choisis dans le groupe consistant en le trioléate, dioléate et monooléate de glycérol, le caprylate de glyceryle, le caprate de glyceryle, le polyglyceryl-2-caprate ainsi que des alcools gras à longues chaînes, linéaires et ramifiés, mono-ou polyhydriques, plus particulièrement l'octyldodécanol, le myristate d'isopropyle et l'octanoate de cétéaryle.

13. Désinfectant selon l'une ou plusieurs des revendications 1 à 12, **caractérisé en ce qu'**il est sous forme de gel.

14. Désinfectant selon l'une ou plusieurs des revendications 1 à 13, **caractérisé en ce qu'**il satisfait la recommandation du Robert Koch Institut pour le contrôle et la déclaration de l'efficacité virucide de désinfectants, et qu'il est efficace dans l'espace d'une minute contre l'adénovirus type 5 (souche adenoid 75), le papovavirus [virus simien 40 (SV40), souche 777], le poliovirus (souche de vaccination contre la polio type I, souche LSc-2ab) et le virus de la vaccine (souche Elstree).

15. Utilisation d'un désinfectant selon l'une ou plusieurs des revendications 1 à 14 pour la désinfection hygiénique de surfaces animées, plus particulièrement de parties de la peau humaine ou animale.

16. Utilisation selon la revendication 15, **caractérisée en ce que** ledit désinfectant est utilisé pour la désinfection hygiénique des mains, la désinfection des mains en chirurgie et après exposition à des virus inconnus.

17. Utilisation selon l'une des revendications 15 ou 16, **caractérisée en ce que** ledit désinfectant est utilisé pour la désinfection des mains en utilisation permanente.

18. Utilisation d'un désinfectant selon l'une ou plusieurs des revendications 1 à 14 pour la désinfection de surfaces inanimées, plus particulièrement de poignées, de cadres de lit, d'instruments chirurgiques, d'équipements d'hôpital ou d'ustensiles de ménage.

19. Utilisation d'un désinfectant selon l'une ou plusieurs des revendications 1 à 14 pour l'éradication de l'adénovirus type 5 (souche adenoid 75), le papovavirus [virus simien 40 (SV40), souche 777], le poliovirus (souche de vaccination contre la polio type I, souche LSc-2ab), le virus de la vaccine (souche Elstree), le virus de la diarrhée virale bovine, et les calicivirus félins.

20. Utilisation d'un désinfectant selon l'une ou plusieurs des revendications 1 à 14 en tant qu'agent bactéricide ou fongicide.

21. Méthode de désinfection, comprenant les étapes consistant à:
a. appliquer un désinfectant selon l'une ou plusieurs des revendications 1 à 14 sur une surface contaminée, et
b. laisser agir le désinfectant pendant une durée suffisante pour l'éradication des virus, champignons et/ou bactéries.

22. Méthode selon la revendication 21, **caractérisée en ce que** l'étape de l'application est réalisée par dépôt par pulvérisation et/ou frottage du désinfectant sur la surface ou par immersion de la surface dans le désinfectant.

23. Méthode selon la revendication 21 ou 22, **caractérisée en ce que** la surface est une peau humaine, plus particulièrement des mains.

24. Produit contenant un désinfectant selon l'une ou plusieurs des revendications 1 à 14.

25. Produit selon la revendication 24, **caractérisé en ce qu'**il est un dispositif d'application par pulvérisation ou un support solide impregné ou traité avec le désinfectant, de préférence des produits textiles plats impregnés, un tissé non tissu en plastique impregné, ou des papiers impregnés.
